# EUROPEAN PATENT APPLICATION

(11) **EP 4 623 801 A1**
(43) Date of publication of application: **01.10.2025**
(21) Application number: 25165506.4
(22) Date of filing: 24.03.2025
(51) Int. Cl.: A61B 1/06, A61B 1/07, A61B 1/04

(54) **ENDOSCOPIC IMAGING SYSTEM AND LIGHT SOURCE THEREOF**

(30) Priority: 25.03.2024 CN 202410346316
(71) Applicant: Wuhan Mindray Bio-Medical Scientific Co., Ltd., Wuhan City Hubei 430206 (CN); Shenzhen Mindray Bio-Medical Electronics Co., Ltd., Shenzhen, Guangdong 518057 (CN)
(72) Inventor: WEI, Xuli, Wuhan, 430206 (CN); LIU, Bin, Shenzhen, 518057 (CN); LI, Chaoran, Wuhan, 430206 (CN); YUAN, Xiaowen, Wuhan, 430206 (CN)
(74) Representative: KIPA AB

(57) **Abstract**

Provided are an endoscopic imaging system and its light source. The light source includes a laser light source, a visible light source and a light combiner; the laser light source outputs laser lights of at least two different wavelengths, wherein the laser lights of different wavelengths excites different fluorescent dyes; the visible light source outputs a visible light; the light combiner combines the laser light(s) and the visible light to form and output a combined beam to an endoscope. The laser light source is a single light-emitting device including a single substrate and at least two laser chips arranged on said substrate; the at least two laser chips emit laser lights of different wavelengths. This light source is capable of exciting different fluorescent dyes in a time-division manner or a simultaneous manner, thus making a layout of the endoscopic imaging system simple and highly reliable, by using the light source.

## Description

### CROSS-REFERENCE TO RELATED APPLICATIONS

This disclosure claims priority to Chinese application No. 202410346316.X, filed on March 25, 2024, to Chinese Patent Office, titled "ENDOSCOPIC IMAGING SYSTEM AND LIGHT SOURCE THEREOF"; the contents of which are incorporated herein by reference in their entirety.

### TECHNICAL FIELD

The disclosure relates to a field of medical device, and in particular to an endoscopic imaging system and a light source thereof.

### BACKGROUND

In recent years, a development of endoscope technology has brought great convenience to minimally invasive surgery, and accelerated popularization of minimally invasive surgery. Traditional reflective endoscope of white light can enter different human tissues and provide visual observation for a doctor with minimal surgical trauma. However, due to limitations in image resolution and contrast, traditional endoscopes are still unable to distinguish subtle lesions that cannot be identified by naked eyes. The emergence of fluorescence endoscope technology has made it possible to visualize these lesions and mark tumours, while also enabling lymphatic localization and vascular tracing.

However, currently most fluorescence endoscopes can only image a single near-infrared fluorescent dye of indocyanine green (ICG), and its poor specificity limits its application in different clinical surgeries. Due to significant differences in excitation and emission wavelength of different fluorescent dyes, current fluorescence endoscopic imaging device is generally not compatible with fluorescence imaging of multiple different wavelengths. In addition, although there are currently a few schemes involving endoscopic imaging of multispectral fluorescence, however, such schemes generally require setting up multiple light sources to emit narrowband lights of different bands, or using a light-emitting device of broad-spectrum, combined with mechanical rotating portions to drive a filter to achieve time-division switching of narrowband spectrum, resulting in high structural complexity and poor reliability.

### SUMMARY

A series of simplified concepts are introduced into summary of this disclosure, and are further elaborated in specific embodiments of this disclosure. The summary of this disclosure does not attempt to define key and necessary technical characteristics of the claimed technical solution, nor attempt to determine a protection scope of the claimed technical solution.

A first aspect according to an embodiment of this disclosure provides an endoscopic imaging system including a light source, an endoscope, an imaging host, and a display;
wherein the light source includes a laser light source, a visible light source, and a light combiner; the laser light source is configured to output laser lights of at least two different wavelengths, wherein the laser lights of different wavelengths are configured to excite different fluorescent dyes; the visible light source is configured to output a visible light; the light combiner is configured to combine the laser light(s) and the visible light to form a combined beam and output the combined beam to the endoscope;
the endoscope includes an insertion portion and an operation portion; wherein the insertion portion is inserted into a part of a patient which part is to be observed, the endoscope is configured to transmit the combined beam to a target object at said part, which target object contains at least one fluorescent dye, and receive a reflected light and a fluorescent light from the target object, and generate an electrical signal;
the imaging host is connected with the endoscope, and configured to separate a reflected light signal and at least one fluorescent light signal from the electrical signal, generate image data of visible light based on the reflected light signal, and generate image data of fluorescent light based on the fluorescent light signal;
the display is connected with the imaging host, and configured to display at least one of a visible light image and a fluorescent light image, based on the image data of visible light and the image data of fluorescent light;
wherein, the laser light source is a single light-emitting device, which includes a single substrate, and at least two laser chips which are arranged on the single substrate; wherein the at least two laser chips are configured to emit the laser lights of different wavelengths, the single light-emitting device further includes a single light outlet and a single optical fiber; wherein the laser light emitted by one of the at least two laser chips or the laser lights emitted by the at least two laser chips is or are outputted from the single light outlet to the single optical fiber.

In an embodiment, the single light-emitting device further includes a single power supply port, which is configured to supply power to the at least two laser chips.

In an embodiment, the light source further includes a collimator, which is arranged between the visible light source and the light combiner, and configured to collimate the visible light which is outputted from the visible light source and transmit the collimated visible light to the light combiner.

In an embodiment, the light source further includes a beam expander, which is arranged between the laser light source and the light combiner, and configured to expand the laser light(s) outputted from the laser light source and transmit the expanded laser light(s) to the light combiner.

In an embodiment, the laser light(s) outputted from the laser light source includes(include) a laser light which is in a near-infrared band, and/or a laser light which is in an ultraviolet band.

In an embodiment, the light combiner includes a filter, wherein the wavelength(s) of the laser light(s) is(are) outside a transmission range of the filter, and the filter is configured to transmit the visible light and reflect the laser light(s), so as to combine the laser light(s) and the visible light to form the combined beam.

In an embodiment, the laser light(s) outputted from the laser light source includes(include) a laser light which is in a near-infrared band, and the filter includes a low-pass filter.

In an embodiment, the wavelength(s) of the laser light(s) outputted from the laser light source is(are) 780nm and/or 660nm, and the transmission range of the filter is below 650nm.

In an embodiment, the laser lights outputted from the laser light source include a laser light which is in a near-infrared band and a laser light which is in an ultraviolet band; wherein the filter includes a bandpass filter.

In an embodiment, the wavelengths of the laser lights outputted from the laser light source include 410nm and at least one of 780nm and 660nm; wherein the transmission range of the filter is 420nm to 650nm.

In an embodiment, the light combiner includes a filter, a dichroic mirror, or a light prism.

In an embodiment, the imaging host is further configured to transmit to the laser light source a power adjustment instruction for a laser light of a target wavelength among the laser lights of at least two different wavelengths;
the laser light source is further configured to adjust power of the laser light of the target wavelength, according to the power adjustment instruction.

In an embodiment, the endoscope includes an optical splitter, a first filter, a second filter, a first image sensor, and a second image sensor, wherein:
the optical splitter is configured to split the reflected light and the fluorescent light into a first path of light and a second path of light; wherein the first path of light includes respective visible light components in the reflected light and the fluorescent light, and the second path of light includes a non-visible light component in the fluorescent light;
the first filter is configured to filter the first path of light, so as to obtain a visible light to be processed;

The second filter is configured to filter the second path of light, so as to obtain a non-visible light to be processed;
the first image sensor is configured to output a visible light signal based on the visible light to be processed;
the second image sensor is configured to output a non-visible light signal based on the non-visible light to be processed;
the imaging host is configured to obtain the reflected light signal based on the visible light signal, and to obtain the fluorescent light signal based on the visible light signal and the non-visible light signal.

Another aspect according to an embodiment of this disclosure provides a light source for an endoscopic imaging system; wherein the light source includes a laser light source, a visible light source, and a light combiner; the laser light source is configured to output laser lights of at least two different wavelengths, wherein the laser lights of different wavelengths are configured to excite different fluorescent dyes; the visible light source is configured to output a visible light; the light combiner is configured to combine the laser light(s) and the visible light to form a combined beam and output the combined beam to an endoscope;
wherein, the laser light source is a single light-emitting device, which includes a single substrate, and at least two laser chips which are arranged on the single substrate; wherein the at least two laser chips are configured to emit laser lights of different wavelengths, the single light-emitting device further includes a single light outlet and a single optical fiber; wherein the laser light emitted by one of the at least two laser chips or the laser lights emitted by the at least two laser chips is or are outputted from the single light outlet to the single optical fiber.

The endoscopic imaging system, according to an embodiment of this disclosure, uses a single light-emitting device to emit laser lights of different wavelengths, which are capable of exciting different fluorescent dyes in a time-division manner or a simultaneous manner, thus making a layout of the light source simple and highly reliable.

### BRIEF DESCRIPTION OF THE DRAWINGS

In order to provide a clearer explanation of technical solutions in embodiments of this disclosure, a brief introduction is given to the accompanying drawings required in the description of the embodiments. It is evident that the accompanying drawings in the following description are only some embodiments of this disclosure. For ordinary technical personnel in the art, other accompanying drawings can be obtained based on these drawings without any creative effort.
FIG. 1 is a structural diagram of an endoscopic imaging system according to an embodiment of this disclosure.
FIG. 2 is a diagram of a light source according to an embodiment of this disclosure.
FIG. 3 is a diagram of a laser light source according to an embodiment of this disclosure.
FIG. 4 is a structural diagram of an endoscope according to an embodiment of this disclosure.

### DESCRIPTION OF THE PREFERRED EMBODIMENTS

In order to make the purpose, technical solution, and advantages of this disclosure more obvious, the following refers to the attached drawings to describe in detail the exemplary embodiments according to this disclosure. Obviously, the described embodiments are only some of the embodiments of this disclosure, not all of them. It should be understood that this disclosure is not limited by the embodiments described here. Based on the embodiments described in this disclosure, all other embodiments obtained by those skilled in the art without creative work, fall within the protection scope of this disclosure.

In the following description, a large number of specific details are provided to provide a more thorough understanding of this disclosure. However, it is evident to those skilled in the art that this disclosure can be implemented without the need for one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art.

The purpose of the terminology used here is only to describe specific embodiments and is not a limitation of this disclosure. When used here, the singular forms of "an", "a", and "the/said" are also intended to include the plural form, unless the context clearly indicates another way. It should also be understood that the terms, such as "including", "and/or", "comprising", when used in this disclosure, determine the presence of the characteristics, integers, steps, operations, components, and/or elements, but do not exclude the presence or addition of one or more other characteristics, integers, steps, operations, components, and/or elements. When used here, the term "and/or" includes any and all combinations of related listed items.

In order to fully understand this disclosure, a detailed structure is provided in the following description to explain the technical solution proposed in this disclosure. The optional embodiments of this disclosure are described in detail below, however, in addition to these detailed description, this disclosure may also have other embodiments.

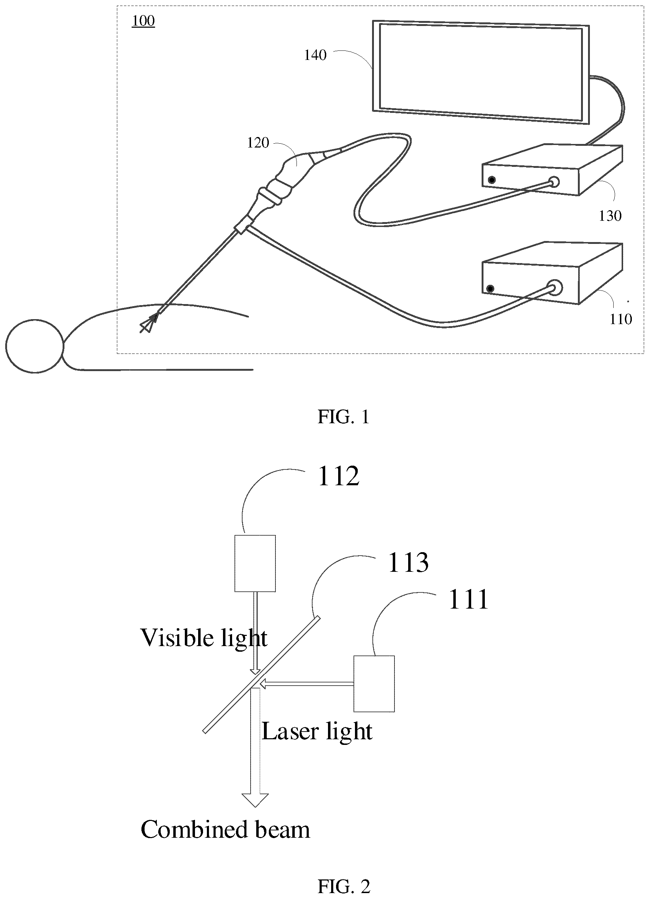

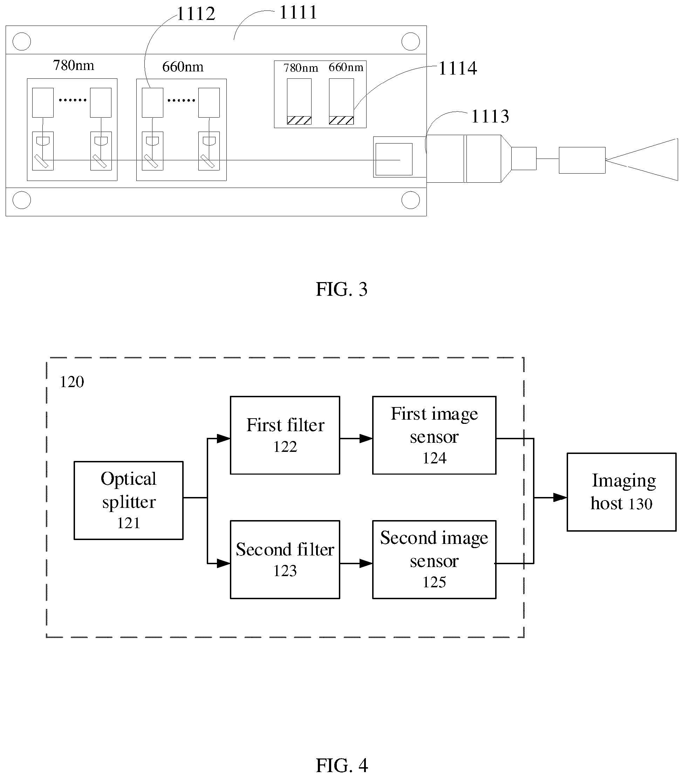

Below, referring to FIGS. 1 to 4, an endoscopic imaging system according to an embodiment of this disclosure is described. FIG. 1 is a structural diagram of an endoscopic imaging system according to an embodiment of this disclosure. FIG. 2 is a diagram of a light source according to an embodiment of this disclosure. FIG. 3 is a diagram of a laser light source according to an embodiment of this disclosure. FIG. 4 is a structural diagram of an endoscope according to an embodiment of this disclosure.

As shown in FIG. 1, the endoscopic imaging system 100 includes a light source 110, an endoscope 120, an imaging host 130, and a display 140. As shown in FIG. 2, the light source 110 includes a laser light source 111, a visible light source 112, and a light combiner 113. The laser light source 111 is configured to output laser lights of at least two different wavelengths, wherein the laser lights of different wavelengths are configured to excite different fluorescent dyes. The visible light source 112 is configured to output a visible light. The light combiner 113 is configured to combine the laser light(s) and the visible light to form a combined beam and output the combined beam to the endoscope 120.

The endoscope 120 includes an insertion portion and an operation portion. The insertion portion is inserted into a part of a patient which part is to be observed. The endoscope is configured to transmit the combined beam to a target object at said part, which target object contains at least one fluorescent dye, receive a reflected light and a fluorescent light from the target object, and generate an electrical signal.

The imaging host 130 is connected with the endoscope 120, and configured to separate a reflected light signal and at least one fluorescent light signal from the electrical signal, generate image data of visible light based on the reflected light signal, and generate image data of fluorescent light based on the fluorescent light signal.

The display 140 is connected with the imaging host 130, and configured to display at least one of a visible light image and a fluorescent light image, based on the image data of visible light and the image data of fluorescent light.

The light source 110 of this embodiment of this disclosure is capable of simultaneously outputting excited lights of different wavelengths which correspond to at least two fluorescent dyes. The endoscope 120 is capable of simultaneously receiving fluorescent lights which correspond to different fluorescent dyes and convert the fluorescent lights into an electrical signal. Fluorescent light signals which correspond to different fluorescent dyes are separated from each other from the electrical signal by the imaging host 130, and one channel of image data of fluorescent light is generated based on each type of fluorescent light signal, thereby achieving multi-channel fluorescence imaging without time division.

The laser light source 111 is capable of simultaneously or separately outputting a narrowband spectrum of multiple different wavelengths for exciting different fluorescent dyes. The visible light source 112 can specifically be a white light source that provides a broadband spectrum, such as a white LED, the visible light provided by the visible light source 112 can be used for reflection imaging of visible light. The light combiner 113 is configured to combine the laser light(s) and the visible light to form a combined beam, allowing the light source 110 to simultaneously output multiple paths of narrowband spectrum and broadband spectrum.

Referring to FIG. 3, the laser light source 111 is a single light-emitting device, which includes a single substrate 1111, and at least two laser chips 1112 which are arranged on the single substrate. The substrate 1111 is provided with a connection terminal and a printed circuit, and the laser chips 1112 are all electrically connected with the substrate 1111.

The at least two laser chips 1112 are configured to emit laser lights of different wavelengths, and a number of the laser chips 1112 which correspond to different wavelengths, can vary depending on a power requirement of sensitivity for a contrast agent.

The single light-emitting device further includes a single light outlet 1113 and a single optical fiber, wherein the laser lights, which are emitted by the at least two laser chips 1112, are outputted from the single light outlet 1113 to the single optical fiber. The imaging host 130 can transmit a control signal to the laser light source 111, so as to control a spectrum of a light which is emitted by the laser light source 111. Each laser light chip 1112 can be individually controlled, specifically, individually controlling includes individually turning on, individually turning off, individually adjusting power. In some embodiments, the imaging host 120 is further configured to transmit to the laser light source a power adjustment instruction for a laser light of a target wavelength among the laser lights of at least two different wavelengths; the laser light source 111 is further configured to adjust power of the laser light of the target wavelength, according to the power adjustment instruction.

When the laser light source 111 emits laser light(s), it can be that one of the laser chips 1112 emits a laser light, or multiple laser chips 1112 are combined to emit laser lights. For example, at least one first laser chip is configured to emit a laser light with a wavelength of 780nm, and at least one second laser chip is configured to emit a laser light with a wavelength of 660nm. The first laser chip and the second laser chip can emit laser lights simultaneously, and the laser lights which are emitted by the laser light source 111 includes two wavelengths of 780nm and 660nm. The first laser chip and the second laser chip can also emit a laser light separately. At this time, the laser light source 111 separately emits a laser light with a wavelength of 780nm, or a laser light with a wavelength of 660nm wavelength.

In an embodiment, the laser light source 111 further includes an optical path channel and a housing, wherein a single light outlet 1113 is arranged at the housing. The light outlet 1113 can be an outlet for an optical fiber. The optical path channel is located between the laser light chip 1112 and the light outlet 1113, and is configured to transmit the laser light which is emitted by the laser light chip 1112 to the light outlet 1113. For example, multiple laser chips 1112 can emit lights in a same direction, and each laser light chip 1112 is equipped with an optical device on its output direction to change a direction of a laser light which is emitted by said laser light chip 1112, so as to conduct said laser light along the optical path channel to the light outlet 1113.

In an embodiment, a photodetector (monitoring PD) is also arranged on the substrate 1111, so as to monitor power of the laser light which is emitted by the laser light chip 1112. For example, a laser light of each wavelength corresponds to one photodetector 1114, and one filter is placed in front of each photodetector 1114 to filter out a laser light of a corresponding wavelength and provide said laser light to the photodetector for monitoring. For example, a photodetector may include a first photodetector which corresponds to a wavelength of 780nm, and a second photodetector which corresponds to a wavelength of 660nm. A 780nm single-pass filter is provided at a front end of the first photodetector to filter out a laser light with a wavelength of 780nm, so that the first photodetector can monitor power of the laser light with a wavelength of 780nm. A 660nm single-pass filter is provided at a front end of the second photodetector to filter out a laser light with a wavelength of 660nm, so that the second photodetector can monitor power of the laser light with a wavelength of 660nm.

For example, a transparent cover can also be provided on the substrate 1111, which encapsulates multiple laser chips 1112 together, thus providing physical protection for the laser chips 1112 and preventing dust or dirt from entering the laser chips 1112, which dust or dirt is inside the light source 110.

For example, the laser light source 111 further includes a single power supply port, and the light source 110 is configured to provide centralized power supply to at least two laser chips through the single power supply port, thereby simplifying the power supply circuit and improving reliability.

The laser light source 111 of the embodiment of this disclosure integrates multiple laser chips 1112 into one light-emitting device, which enables the laser light source 111 to have good scalability and to emit laser lights of at least two wavelengths separately or simultaneously, without requiring setting a coupling optical path to couple laser lights of different wavelengths, simplifying the optical path structure and reducing costs.

For example, the light source 110 further includes a heat dissipation component which is configured to provide heat dissipation for the laser light source. The heat dissipation component includes but is not limited to a thermoelectric cooler (TEC), which directly uses an electric thermal conversion to achieve a cooling purpose and a precise temperature control. Due to the fact that the laser light source is a single light-emitting device, it is conducive to centralized heat dissipation management of the light source, making an internal layout of the light source simple and easy to integrate.

The light source 110 further includes a combiner 113, which is configured to combine the laser light(s) which is(are) outputted from the laser light source 111 and the visible light which is outputted from the visible light source 112 to form a combined beam, and output the combined beam to the endoscope 120. The endoscopic imaging system 100 requires a visible light to provide a field of view, and to provide navigation for a fluorescent light, during fluorescence imaging. Therefore, while the laser light source 111 provides an exciting light, the visible light source 112 also needs to provide the visible light, both the exciting light and the visible light are combined and outputted through the light combiner 113. Due to the fact that the laser light source 111 in the embodiment of this disclosure is a single light-emitting device, laser lights of different wavelengths are outputted through the same light outlet 1113, thus simplifying a structure and a light path of the light combiner 113.

In an embodiment, as shown in FIG. 2, the light combiner 113 can be a filter, such as a filtering sheet, a dichroic mirror, or a light prism. A wavelength of the laser light is outside a transmission range of the filter, wherein the filter transmits the visible light and reflects the laser light, so as to combine the laser light and the visible light to form a combined beam. The filter is tilted and arranged between the laser light source 111 and the visible light source 112, and has characteristics of semi-transparent and semi-reflective. Due to that the wavelength of the laser light is outside the transmission range of the filter, a direction of an optical path of the laser light, which is irradiated on a surface of the filter, changes. The visible light is a wideband light, and most of the visible light is transmitted by the filter without changing its direction. After being reflected and transmitted by the filter, the laser light and the visible light become in the same direction, so as to form the combined beam.

In a specific example, the laser light(s) outputted from the laser light source, includes(include) a laser light which is in a near-infrared band, and the filter can be a low-pass filter, which can only transmit a light which is below a certain wavelength. Specifically, a filter is unable to transmit a laser light in a near-infrared band, so as to change the direction of the optical path of the laser light.

For example, when wavelengths of the laser lights, which are outputted from the laser light source, are 780nm and 660nm, fluorescence imaging of MB (methylene blue) and ICG (indocyanine green), are respectively supported. Correspondingly, the transmission range of the filter is low than 650nm. The laser lights with wavelengths of 780nm and 660nm cannot be transmitted by a filter, but can only be reflected on its surface, so as to be combined with the transmitted visible light, so as to form the combined beam.

In another example, the laser light(s) outputted from the laser light source includes(include) a laser light which is in a near-infrared band and/or a laser light which is in an ultraviolet band, and the filter includes a bandpass filter, which can only transmit a light within a certain wavelength range. The laser light in the near-infrared band and the laser light in the ultraviolet band are located outside the transmission range of the bandpass filter, and therefore cannot be transmitted by the filter.

For example, wavelengths of near-infrared laser lights are 780nm and 660nm respectively, and a wavelength of an ultraviolet laser light is 410nm, which lights support fluorescence imaging of MB (methylene blue), ICG (indocyanine green), and PPIX. Correspondingly, a transmission range of the filter is 420nm to 650nm. The wavelengths of 780nm and 660nm are larger than the transmission range of the filter, while the wavelength of 410nm is smaller than the transmission range of the filter. Accordingly, these wavelengths cannot be transmitted by the filter and can only be reflected on its surface, so as to be combined with the transmitted visible light to form the combined beam. Specifically, when it is necessary to simultaneously output both laser lights of the near-infrared band and of the ultraviolet band; the laser lights, which are outputted from the laser light source, can be configured as 780nm and 420nm respectively, or 660nm and 420nm, respectively, or 420nm, 660nm, and 780nm, respectively. The light source configuration can be completed according to a selection instruction from a user, so as to achieve precise output of laser light, and reduce light interference and noise.

In some embodiments, the light source 110 further includes a beam expander, which is arranged between the laser light source 111 and the light combiner 113, for expanding the laser light(s) which is(are) outputted from the laser light source, and configured to expand the laser light(s) outputted from the laser light source and transmit the expanded laser light(s) to the light combiner. A beam diameter of the laser light which is outputted from the laser light source is relatively small. After passing through the beam expander, the beam diameter of the laser light can be expanded to match a beam diameter of the visible light, thereby improving a beam combining effect. For example, the beam expander may include a lens group, a diffuser plate, etc.

In some embodiments, the light source 110 further includes a collimator, which is arranged between the visible light source 112 and the light combiner 113, and configured to collimate the visible light which is outputted from the visible light source 112 and transmit the collimated visible light to the light combiner. A beam of the visible light which is outputted by the visible light source 112 is relatively divergent, and the divergent light can be converted into a parallel light through a collimator to improve the beam combining effect. For example, the collimator may include a lens group, a light guide rod, etc.

The endoscope 120 is configured to receive the combined beam which is outputted by the light combiner 113. For example, the endoscope 120 includes an insertion portion and an operation portion. The insertion portion is inserted into a part of a patient which part is to be observed, and the operation portion is used for a handheld operation of a user. The insertion portion and the operation portion can be an integrated structure or a detachable structure. The endoscope further includes at least one image sensor (not shown), for example, the image sensor may be provided at a front end of the insertion portion of the endoscope. The endoscope 120 is capable of transmitting the combined beam to a target object which contains at least one fluorescent dye, and receive a reflected light and a fluorescent light from the target object, and convert the reflected light and the fluorescent light into an electrical signal through an image sensor.

The other end of endoscope 120 is connected with an imaging host 130 through a cable, so as to transmit the electrical signal to the imaging host 130 for processing. **In** some embodiments, the endoscope may also transmit the electrical signal wirelessly to the imaging host 130. The imaging host 130 is configured to separate a reflected light signal and at least one fluorescent light signal from the electrical signal, generate image data of visible light based on the reflected light signal, and generate image data of fluorescent light based on the fluorescent light signal. Specifically, the imaging host 130 is provided with a processor which obtains the electrical signal which is outputted by the endoscope 120 and generates the image data of visible light and the image data of fluorescent light.

In an embodiment, as shown in FIG. 4, the endoscope 120 includes an optical splitter 121, a first filter 122, a second filter 123, a first image sensor 124, and a second image sensor 125. The optical splitter 121 is configured to split the reflected light and the fluorescent light into a first path of light and a second path of light. The first path of light includes respective visible light components in the reflected light and the fluorescent light, and the second path of light includes a non-visible light component in the fluorescent light; The first filter 122 is configured to filter the first path of light, so as to obtain a visible light to be processed; the second filter 123 is configured to filter the second path of light, so as to obtain a non-visible light to be processed. The first image sensor 124 is configured to output a visible light signal based on the visible light to be processed; the second image sensor 125 is configured to output a non-visible light signal based on the non-visible light to be processed. The first image sensor 124 is an image sensor of visible light, and the second image sensor 125 is an image sensor of non-visible light.

The imaging host 130 is configured to obtain the reflected light signal based on the visible light signal, and to obtain the fluorescent light signal based on the visible light signal and the non-visible light signal. Due to that the visible light component in the fluorescent light is contained in the first path of light and the non-visible light component is contained in the second path of light, a complete fluorescent light signal can be obtained by combining the visible light signal and the non-visible light signal. The imaging host 130 is further configured to generate the image data of visible light based on the reflected light signal, and to generate the image data of fluorescent light based on the fluorescent light signal.

The display 140 is connected with the imaging host 130 and configured to display at least one of a visible light image and a fluorescent light image, based on the image data of visible light and the image data of fluorescent light. Specifically, the imaging host 130 is connected with the display 140 through a video cable for transmitting an endoscopic image to the display 140 for display.

When the target object contains at least two types of fluorescent dyes, and the imaging host separates fluorescent light signals which correspond to the at least two types of fluorescent dyes, the display 140 can overlay or fuse at least two types of fluorescent images for displaying, and the different types of fluorescent images are distinguished by different colours. Alternatively, the display 140 can display at least two types of fluorescent images separately. The at least two types of fluorescent images can be overlaid on a visible light image for display.

It should be noted that FIG. 1 is only an example of the endoscopic imaging system 100 and does not constitute a limitation on the endoscopic imaging system 100. The endoscopic imaging system 100 may include more or fewer components than shown in FIG. 1, or combine certain components, or include different components. For example, the endoscopic system 100 may also include a dilator, a smoke control device, an input/output device, a network access device, etc.

In summary, the endoscopic imaging system 100 of this disclosure uses a single light-emitting device to emit laser lights of different wavelengths, which are capable of exciting different fluorescent dyes in a time-division manner or a simultaneous manner, thus making a layout of the light source simple and highly reliable.

Another aspect according to an embodiment of this disclosure provides a light source for an endoscopic imaging system; wherein the light source includes a laser light source, a visible light source, and a light combiner; the laser light source is configured to output laser lights of at least two different wavelengths, wherein the laser lights of different wavelengths are configured to excite different fluorescent dyes; the visible light source is configured to output a visible light; the light combiner is configured to combine the laser light(s) and the visible light to form a combined beam and output the combined beam to an endoscope;
wherein, the laser light source is a single light-emitting device, which includes a single substrate, and at least two laser chips which are arranged on the single substrate; wherein the at least two laser chips are configured to emit laser lights of different wavelengths, the single light-emitting device further includes a single light outlet and a single optical fiber; wherein the laser light emitted by one of the at least two laser chips or the laser lights emitted by the at least two laser chips is or are outputted from the single light outlet to the single optical fiber.

In an embodiment, the single light-emitting device further includes a single power supply port, which is configured to supply power to the at least two laser chips.

In an embodiment, the light source further includes a collimator, which is arranged between the visible light source and the light combiner, and configured to collimate the visible light which is outputted from the visible light source and transmit the collimated visible light to the light combiner.

In an embodiment, the light source further includes a beam expander, which is arranged between the laser light source and the light combiner, and configured to expand the laser light(s) outputted from the laser light source and transmit the expanded laser light(s) to the light combiner.

In an embodiment, the laser light(s) outputted from the laser light source includes(include) a laser light which is in a near-infrared band, and/or a laser light which is in an ultraviolet band.

In an embodiment, the light combiner includes a filter, wherein the wavelength of the laser light is outside a transmission range of the filter, and the filter is configured to transmit the visible light and reflect the laser light(s), so as to combine the laser light(s) and the visible light to form the combined beam.

In an embodiment, the laser light(s) outputted from the laser light source includes(include) a laser light which is in a near-infrared band, and the filter includes a low-pass filter. For example, the wavelength(s) of the laser light(s) outputted from the laser light source is(are) 780nm and/or 660nm, and the transmission range of the filter is below 650nm.

In an embodiment, the laser lights outputted from the laser light source include a laser light which is in a near-infrared band and a laser light which is in an ultraviolet band; wherein the filter includes a bandpass filter. For example, the wavelengths of the laser lights outputted from the laser light source include 410nm and at least one of 780nm and 660nm; wherein the transmission range of the filter is 420nm to 650nm.

In an embodiment, the light combiner includes a filter, a dichroic mirror, or a light prism.

The light source of this embodiment of this disclosure uses a single light-emitting device to emit laser lights of different wavelengths, which are capable of exciting different fluorescent dyes in a time-division manner or a simultaneous manner, thus making a layout of the light source simple and highly reliable. More specific details about the light source can be found in the endoscopic imaging system 100 mentioned earlier, which will not be elaborated here.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some characteristics can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Ordinary technical personnel in this field can understand that, except mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

In addition, Ordinary technical personnel in this field can understand that although some embodiments described herein include certain characteristics rather than other characteristics included in other embodiments, the combination of characteristics of different embodiments means that they are within the scope of this disclosure and form different embodiments. For example, in the claims, any one of the claimed embodiments can be used in any combination.

The various component embodiments of this disclosure can be implemented in hardware, software modules running on one or more processors, or a combination thereof. Ordinary technical personnel in this field should understand that a microprocessor or digital signal processor (DSP) can be used in practice to implement some or all of the functions of some modules according to the embodiments of this disclosure. This disclosure can also be implemented as a partial or complete device program (such as a computer program and a computer program product) for executing the method described herein. The program implementing this disclosure can be stored at a computer-readable medium or can have the form of one or more signals. Such signals can be downloaded from internet websites, provided on carrier signals, or in any other form.

It should be noted that the above embodiments illustrate this disclosure rather than limit it, and Ordinary technical personnel in this field can design alternative embodiments without departing from the scope of the attached claims. **In** the claims, any reference symbol between parentheses should not be constructed as a restriction on the claims. This disclosure can be implemented with the help of hardware consisting of several different components and with the help of appropriately programmed computers. Among the unit claims that list several devices, several of these devices can be specifically embodied through the same hardware item. The use of words first, second, and third does not indicate any order. These words can be interpreted as names.

The above is only the specific implementation method or explanation of the specific implementation method of this disclosure. The protection scope of this disclosure is not limited to this. Any technical personnel familiar with the technical field can easily think of changes or replacements within the technical scope disclosed in this disclosure, and those changes or replacements should fall into the protection scope of this disclosure. The protection scope of this disclosure shall be based on the protection scope of the claims.

## Claims

1. An endoscopic imaging system, **characterized in that**, comprising a light source, an endoscope, an imaging host, and a display;
wherein the light source comprises a laser light source, a visible light source, and a light combiner; the laser light source is configured to output laser lights of at least two different wavelengths, wherein the laser lights of different wavelengths are configured to excite different fluorescent dyes; the visible light source is configured to output a visible light; the light combiner is configured to combine the laser light(s) and the visible light to form a combined beam and output the combined beam to the endoscope;
the endoscope comprises an insertion portion and an operation portion; wherein the insertion portion is inserted into a part of a patient which part is to be observed, the endoscope is configured to transmit the combined beam to a target object at said part, which target object contains at least one fluorescent dye, and receive a reflected light and a fluorescent light from the target object, and generate an electrical signal;
the imaging host is connected with the endoscope, and configured to separate a reflected light signal and at least one fluorescent light signal from the electrical signal, generate image data of visible light based on the reflected light signal, and generate image data of fluorescent light based on the fluorescent light signal;
the display is connected with the imaging host, and configured to display at least one of a visible light image and a fluorescent light image, based on the image data of visible light and the image data of fluorescent light;
wherein, the laser light source is a single light-emitting device, which comprises a single substrate and at least two laser chips which are arranged on the single substrate; wherein the at least two laser chips are configured to emit the laser lights of different wavelengths, the single light-emitting device further comprises a single light outlet and a single optical fiber; wherein the laser light emitted by one of the at least two laser chips or the laser lights emitted by the at least two laser chips is or are outputted from the single light outlet to the single optical fiber.

2. The endoscopic imaging system according to claim 1, **characterized in that**, the single light-emitting device further comprises a single power supply port, which is configured to supply power to the at least two laser chips.

3. The endoscopic imaging system according to claim 1, **characterized in that**, the light source further comprises a collimator, which is arranged between the visible light source and the light combiner, and configured to collimate the visible light which is outputted from the visible light source and transmit the collimated visible light to the light combiner.

4. The endoscopic imaging system according to claim 2 or 3, **characterized in that**, the light source further comprises a beam expander, which is arranged between the laser light source and the light combiner, and configured to expand the laser light(s) outputted from the laser light source and transmit the expanded laser light(s) to the light combiner.

5. The endoscopic imaging system according to claim 1, **characterized in that**, the laser light(s) outputted from the laser light source comprises(comprise) a laser light which is in a near-infrared band, and/or a laser light which is in an ultraviolet band.

6. The endoscopic imaging system according to claim 1, **characterized in that**, the light combiner comprises a filter, wherein the wavelength(s) of the laser light(s) is(are) outside a transmission range of the filter, and the filter is configured to transmit the visible light and reflect the laser light(s), so as to combine the laser light(s) and the visible light to form the combined beam.

7. The endoscopic imaging system according to claim 6, **characterized in that**, the laser light(s) outputted from the laser light source comprises(comprise) a laser light which is in a near-infrared band, and the filter comprises a low-pass filter.

8. The endoscopic imaging system according to claim 7, **characterized in that**, the wavelength(s) of the laser light(s) outputted from the laser light source is(are) 780nm and/or 660nm, and the transmission range of the filter is below 650nm.

9. The endoscopic imaging system according to claim 6, **characterized in that**, the laser lights outputted from the laser light source comprise a laser light which is in a near-infrared band and a laser light which is in an ultraviolet band; wherein the filter comprises a bandpass filter.

10. The endoscopic imaging system according to claim 9, **characterized in that**, the wavelengths of the laser lights outputted from the laser light source comprise 410nm and at least one of 780nm and 660nm; wherein the transmission range of the filter is 420nm to 650nm.

11. The endoscopic imaging system according to any one of claims 6-10, , **characterized in that**, the light combiner comprises a filter, a dichroic mirror, or a light prism.

12. The endoscopic imaging system according to claim 1, **characterized in that**, the imaging host is further configured to transmit to the laser light source a power adjustment instruction for a laser light of a target wavelength among the laser lights of at least two different wavelengths;
the laser light source is further configured to adjust power of the laser light of the target wavelength, according to the power adjustment instruction.

13. The endoscopic imaging system according to claim 1, **characterized in that**, the endoscope comprises an optical splitter, a first filter, a second filter, a first image sensor, and a second image sensor, wherein:
the optical splitter is configured to split the reflected light and the fluorescent light into a first path of light and a second path of light; wherein the first path of light comprises respective visible light components in the reflected light and the fluorescent light, and the second path of light comprises a non-visible light component in the fluorescent light;
the first filter is configured to filter the first path of light, so as to obtain a visible light to be processed;
the second filter is configured to filter the second path of light, so as to obtain a non-visible light to be processed;
the first image sensor is configured to output a visible light signal based on the visible light to be processed;
the second image sensor is configured to output a non-visible light signal based on the non-visible light to be processed;
the imaging host is configured to obtain the reflected light signal based on the visible light signal, and to obtain the fluorescent light signal based on the visible light signal and the non-visible light signal.

14. A light source for an endoscopic imaging system; **characterized in that**, the light source comprises a laser light source, a visible light source, and a light combiner; the laser light source is configured to output laser lights of at least two different wavelengths, wherein the laser lights of different wavelengths are configured to excite different fluorescent dyes; the visible light source is configured to output a visible light; the light combiner is configured to combine the laser light(s) and the visible light to form a combined beam and output the combined beam to an endoscope;
wherein, the laser light source is a single light-emitting device, which comprises a single substrate, and at least two laser chips which are arranged on the single substrate; wherein the at least two laser chips are configured to emit laser lights of different wavelengths, the single light-emitting device further comprises a single light outlet and a single optical fiber; wherein the laser light emitted by one of the at least two laser chips or the laser lights emitted by the at least two laser chips is or are outputted from the single light outlet to the single optical fiber.

15. The light source according to claim 14, **characterized in that**,
the laser light(s) outputted from the laser light source comprises(comprise) a laser light which is in a near-infrared band, and/or a laser light which is in an ultraviolet band; or
the light combiner comprises a filter, wherein the wavelength(s) of the laser light(s) is(are) outside a transmission range of the filter, and the filter is configured to transmit the visible light and reflect the laser light(s), so as to combine the laser light(s) and the visible light to form the combined beam.
